# EUROPEAN PATENT APPLICATION

(11) **EP 3 862 050 A1**
(43) Date of publication of application: **11.08.2021**
(21) Application number: 20156042.2
(22) Date of filing: 07.02.2020
(51) Int. Cl.: A61Q 5/00, A61Q 17/04, A61Q 19/00, A61K 8/365, A61K 8/368, A61K 8/37, A61K 8/49, A61K 8/60, A61K 8/9789, A61P 17/00, A61P 17/06, A61P 17/10, A61P 17/16, A61P 17/18, A61Q 19/08

(54) **LITCHI PRODUCTS AS DERMATOLOGICAL AND COSMETIC AGENTS**

(71) Applicant: Dr. August Wolff GmbH & Co. KG Arzneimittel, 33611 Bielefeld (DE)
(72) Inventor: Soeberdt, Michael, 33611 Bielefeld (DE); Abels, Christoph, 33611 Bielefeld (DE)
(74) Representative: Ter Meer Steinmeister & Partner

(57) **Abstract**

The present invention relates to a litchi product and use thereof as a skin or hair care product, in particular as a cosmetic or as a (dermatological) pharmaceutical agent. The litchi product of the invention is suitable for and effective in restoring the physiological pH of skin or hair and/or in protecting the skin or hair against oxidative stress e.g. caused by light irradiation and other environmental influences. It is effective in the medical and cosmetic treatment and prophylaxis of several skin or hair diseases and conditions. Moreover, the invention relates to a process for preparing the litchi product.

## Description

The present invention relates to a litchi product and use thereof as a skin care product, in particular as a cosmetic or as a (dermatological) pharmaceutical agent. The litchi product of the invention is suitable for and effective in restoring the physiological pH of skin and hair and/or in protecting skin and hair against oxidative stress e.g. caused by light irradiation and other environmental influences. It is effective in the medical and cosmetic treatment and prophylaxis of several skin and hair diseases and conditions, e.g. skin or hair aging. Moreover, the invention relates to a process for preparing the litchi product.

The skin including skin appendages is the largest body organ that regulates excretion of metabolic waste products, temperature, and plays an important role in body protection against environmental factors of all kinds including physical and chemical as well as biological factors. These include agents that may act as oxidants or catalysts of reactions producing reactive oxygen species (ROS), reactive nitrogen species (RNS), and other oxidants in skin cells. An increased amount of the oxidants, exceeding the antioxidant defense system capacity is called oxidative stress, leading to chronic inflammation, which, in turn, can cause collagen fragmentation and disorganization of collagen fibers and skin cell functions, and thus contribute to various skin diseases and conditions including cancer (Kruk J. and Duchnik E. in Asian Pac. J. Cancer Prev. 2014; 15(2):561-8).

The pH of skin and hair is of crucial importance for their proper physiological functioning. The pH of older skin as well as that of diseased skin is known to be higher in comparison with that of young and healthy skin (i.e. the acidity at the skin surface and/or in the stratum corneum is lowered) and therefore physiological skin functions may be impaired. To counteract this problem, it has been proposed that skin care products be formulated with a lower pH for older skin. It is assumed that this would normalize the age-related increase in the pH of skin and allow the physiological skin functions to be maintained (Schreml et al., EMJ Dermatol. 2014; 2:86-94). However, in the acidic pH range topical compositions tend to become instable including galenic/physical instability of the composition *per se* (e.g. phase separation, inhomogeneity of ingredients) and chemical instability of the active ingredients. This usually results in a significant decrease in pharmaceutical and/or cosmetic efficacy. Moreover, skin tolerance, compatibility and patient's/user's convenience are often reduced. In addition, formulations which are stable at low pH often are cosmetically inacceptable. Therefore, it is necessary to develop a formulation that is both stable and cosmetically acceptable.

There is therefore a demand for topical dermatological and cosmetic compositions having improved properties. In particular, there is a need to provide for topical dermatological and cosmetic compositions that will be effective in protecting skin and hair against oxidative stress and can lower the pH of skin quickly and decisively while at the same time being stable even at a low pH.

The object on which the present invention is based thus lies in providing a generally stable topical dermatological or cosmetic composition having improved properties in view of a) protecting skin and hair against oxidative stress caused e.g. by light radiation or other environmental influences and b) correcting age-related or disease-induced disturbance in skin or hair function.

This object has surprisingly been solved according to the subject matter of the present invention as defined in the claims.

The present invention relates to a litchi product for use as a medicament, preferably as a dermatological agent, a medical device and as a cosmetic. The litchi product, preferably being selected from litchi wine and litchi vinegar, is effective in restoring the physiological pH of skin and in protecting skin against oxidative stress. Moreover, it is suitable to be used in the therapeutic and prophylactic treatment of several skin and hair diseases and in the non-therapeutic (cosmetic) treatment of various skin and hair conditions including skin and hair aging.

It has surprisingly been found that the litchi product and the medical/cosmetic compositions comprising the litchi product according to the invention are effective in protecting skin and hair against oxidative stress caused e.g. by light radiation or other environmental influences, and in correcting the age-related or disease-induced disturbance in skin and hair function, while they are very stable even in the acidic pH range. It is assumed that these effects are based on a synergistic action of the active ingredients and the low pH of the litchi product and the compositions comprising the litchi product. In other words, they have a good stability even at a low pH (i.e. both including chemical stability of the active ingredients and galenic/physical stability of the composition with neither phase separation nor inhomogeneity of ingredients). At the same time, the pH on the skin (pHss) as well as that in the stratum corneum (pHsc) can be lowered effectively. Therefore, the litchi product according to the invention makes it possible to effectively restore the physiological properties (such as the epidermal barrier functions and barrier integrity) of older and/or diseased skin in particular, in which the acidity at the skin surface and/or in the stratum corneum is reduced. The reduction in pH on the skin and in the stratum corneum according to the invention is achieved unexpectedly rapidly and distinctly for those skilled in the art. In addition, the litchi product according to the invention makes it possible to effectively improve the appearance of skin and hair (anti-aging), for providing antioxidative effects and/or for increasing the moisture content of skin and hair. As a result, it has surprisingly been found that a plurality of medical and cosmetic indications for skin and hair can be treated effectively with the litchi product and the medical/cosmetic compositions comprising the litchi product according to the invention. At the same time skin tolerance, skin and hair care and protection, compatibility and patient's/user's convenience are significantly increased according to the present invention. In summary, the litchi product of the present invention surprisingly provides for combined antioxidative effects, antibacterial effects, fungicidal effects, anti-inflammatory effects, as well as skin and hair care and skin and hair protection effects rendering it perfectly suitable also for use in anti-aging products. The combination of all these effects was not yet achievable in the prior art.

In accordance with the present invention oxidative stress is understood to be caused by any kind of environmental influence. This influence includes light irradiation, e.g. selected from sunlight (UV such as UVA and UVB, IR and visible light) and artificial light such as display light, and other environmental influences, e.g. temperature, nutrition, stress, lack of sleep, air/water pollution, tobacco, and/or alcohol.

The preferred embodiments of the present invention are described in greater detail below.

Litchi (also: lychee; *Litchi chinensis Sonn*.) is a fruit native to South China, and popular for the taste and health benefits upon oral application. Litchi products obtained from litchi fruits are known to include litchi wine and litchi vinegar (i.e. extracted and fermented from litchi fruit). All kinds of litchi products are commercially available (such as from Guangdong Zhenzhou Litchi Industrial Co., Ltd., Huizhou, China) and suitable to be applied topically as a dermatologic or cosmetic agent according to the present invention. However, litchi vinegar is particularly preferred due to its well-balanced composition of active ingredients and pH value. It was found out according to the present invention that litchi products (such as litchi wine or litchi vinegar) contain as the main active ingredients rutin, ferulic acid, protocatechuic acid (3,4-dihydroxybenzoic acid), epicatechin, chlorogenic acid, caffeic acid, and gallic acid. Most important from the perspective of skin care characteristics were found out to be rutin, ferulic acid, protocatechuic acid (3,4-dihydroxybenzoic acid), epicatechin, and chlorogenic acid. These compounds have the following structural formulae:

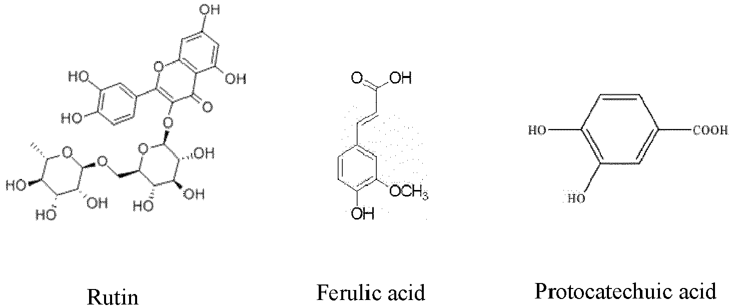

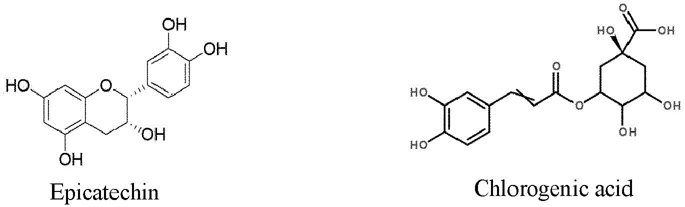

It was determined according to the present invention that e.g. chlorogenic acid functions as an antioxidant and inter alia provides for skin or hair conditioning and skin or hair protection effects. Rutin is an antioxidant and inter alia provides for hair conditioning and skin conditioning effects. Caffeic acid is an antioxidant, and protocatechuic acid (3,4-dihydroxybenzoic acid) is effective in skin protection. Preferably, the litchi product such as litchi vinegar further contains acetic acid in an amount of 3.5% by weight or more based on the weight of the litchi vinegar, more preferably of 3.5 to 10%, even more preferably 5 to 8%, by weight or more based on the weight of the litchi vinegar.

A suitable process for preparation of a litchi product (such as litchi vinegar) comprises the steps of harvesting litchi fruits, fruit sorting applying e.g. infrared technology, washing, peeling, pressing of pulps to obtain litchi juice, freeze-drying of juice, first fermentation, removal of alcohol, second fermentation, optionally followed by analysis of the product. Preferably the litchi vinegar is prepared according to the method disclosed in "National Standard of the People's Republic of China, GB/T 18187-2000, Fermented Vinegar".

As mentioned above, the present invention relates to a topical dermatological or cosmetic composition comprising a litchi product preferably selected from litchi wine and litchi vinegar. The composition can be used as a pharmaceutical composition/medicament, as a medical device or as a cosmetic composition, depending on the indication to be treated and the respective regulatory requirements. The composition comprises the litchi product, preferably the litchi vinegar, in an amount of 0.1 to 10%, preferably 0.5 to 8%, more preferably 0.75 to 5%, even more preferably 1 to 3%, and most preferably about 1%, each by weight based on the total composition. Suitable amounts of litchi product (namely litchi wine, or litchi vinegar; most preferred litchi vinegar) in the topical composition of the invention are 1%, 2%, 3%, 4%, 5%, 6%, 7%, 8%, 9%, or 10%, each by weight based on the total composition. Most preferred is an amount of 1% by weight litchi vinegar based on the total composition.

The topical dermatological or cosmetic composition according to the present invention preferably has an acidic pH of 1 to 6, more preferably of 2 to 5, even more preferably of 3 to 5, most preferably of 4 to 5 (such as pH 4 or pH 5). The pH value of the topical dermatological or cosmetic composition is measured with a pH meter. The topical dermatological or cosmetic composition according to the invention may further comprise a buffering agent/buffer system comprising polyacids or α-hydroxy acids (AHAs) and their respective salts to adjust or stabilize the pH value in the composition as well as following topical application to achieve the above-mentioned effects. Examples for such polyacids are citric acid, tartaric acid and malic acid. Examples of AHAs include glycolic acid, lactic acid and mandelic acid. Most preferred is a citric acid/citrate buffer system.

The topical dermatological or cosmetic composition according to the present invention may contain additional amounts of the active ingredients of litchi in order to further increase the skin care benefits provided. Thus, to the compositions may be added suitable amounts of one or more compounds selected from rutin, ferulic acid, protocatechuic acid, epicatechin, chlorogenic acid, caffeic acid, and gallic acid. Preferably additional amounts of at least one of rutin, ferulic acid, protocatechuic acid, epicatechin, and chlorogenic acid are added. More preferably additional amounts of at least one of ferulic acid, protocatechuic acid, and chlorogenic acid are added. Most preferably chlorogenic acid is added. A suitable source of additional chlorogenic acid is Eucommia ulmoides Oliv. bark extract. Suitable amounts of the compounds are 0.01 to 5%, preferably 0.1 to 3%, more preferably 0.5 to 2%, each by weight based on the total composition, respectively. Particularly preferred is the addition of a combination of ferulic acid, protocatechuic acid, and chlorogenic acid in amounts of 0.01 to 1%, preferably 0.05 to 0.75%, more preferably 0.1 to 0.5%, each per compound of the combination and each by weight based on the total composition. By adding the additional amounts of active ingredients of litchi to the litchi product (in particular to litchi vinegar) there is obtained a synergistic effect of skin and/or hair care benefits.

The topical dermatological or cosmetic composition according to the invention may further comprise a light protecting agent. These include light protectants (such as sun protecting agents/SPF agents and sun blockers) having one or more light filter compounds such as UV filter systems. Preferably they contain UVB filter systems known to the skilled person.

UV filters are selected from the group consisting of UVA filters, UVB filters, and light protection pigments. Generally, UV filters are capable of absorbing ultraviolet radiation and releasing the absorbed energy in the form of long-wave radiation such as heat. According to the invention UV filters are preferably contained in amounts of 0.05% to 50%, preferably 0.5% to 40% and more preferably 5% to 30%, each by weight based on the total composition. Particularly suitable are amounts of 15 to 25%, such as 16%, 17%, 18%, 19%, 20%, 21%, 22%, 23% or 24%, each by weight based on the total composition. UV filters can be oil-soluble or water-soluble. Examples of suitable oil-soluble substances include:
- 3-benzylidene camphor or 3-benzylidene norcamphor and derivatives thereof, such as 3-(4-methylbenzylidene)camphor;
- 4-aminobenzoic acid derivatives, preferably 4-(dimethylamino)benzoic acid-2-ethyl- hexyl ester, 4-(dimethylamino)benzoic acid-2-octyl ester, and 4-(dimethylamino)- benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, and 2-cyano-3,3-phenylcinnamic acid-2-ethylhexyl ester (octocrylene);
- esters of salicylic acid, preferably salicylic acid-2-ethylhexyl ester, salicylic acid-4-isopropyl benzyl ester, and salicylic acid homomenthyl ester;
- benzophenone derivatives, preferably 2-hydroxy-4-methoxybenzophenone, 2- hydroxy-4-methoxy-4'-methylbenzophenone, and 2,2'-dihydroxy-4-methoxybenzo-phenone;
- esters of benzylmalonic acid, preferably 4-methoxybenzylmalonic acid di-2-ethyl-hexyl ester;
- triazine derivatives such as 2,4,6-trianilino-(p-carbo-2'-ethyl-1'-hexyloxy)-1,3,5-triazine and octyl triazone or dioctyl butamidotriazone (Uvasorb® HEB);
- propane-1,3-diones such as 1-(4-tert-butylphenyl)-3-(4'-methoxyphenyl)propane-1,3-dione; and
- ketotricyclo (5.2.1.0) decane derivatives.

Examples of suitable water-soluble substances include:
- 2-phenylbenzimidazole-5-sulfonic acid and alkali, alkaline earth, ammonium, alkylammonium, alkanolammonium, and glucammonium salts thereof;
- 1H-benzimidazole-4,6-disulfonic acid, 2,2'-(1,4-phenylene)bis-disodium salt (Neo Heliopan® AP);
- sulfonic acid derivatives of benzophenones, preferably 2-hydroxy-4-methoxybenzophenone-5-sulfonic acid and salts thereof;
- sulfonic acid derivatives of 3-benzylidene camphor such as 4-(2-oxo-3-bornylidene methyl)benzene sulfonic acid, 2-methyl-5-(2-oxo-3-bornylidene)sulfonic acid, and salts thereof.

Typical examples of particularly suitable UVA filters include benzoyl methane derivatives such as 1-(4'-tert-butylphenyl)-3-(4'-methoxyphenyl)propane-1,3-dione, 4-tert-butyl-4'-methoxy-dibenzoyl methane (Parsol® 1789), 2-(4-diethylamino-2-hydroxybenzoyl)-benzoic acid hexyl ester (Uvinul® A Plus), 1-phenyl-3-(4'-isopropylphenyl)-propane-1,3-dione, as well as enamine compounds. The UVA and UVB filters can also be used in mixtures. Particularly suitable combinations comprise (or even consist of) benzoyl methane derivatives such as 4-tert-butyl-4'-methoxydibenzoyl methane (Parsol® 1789) and 2-cyano-3,3-phenylcinnamic acid-2-ethyl-hexyl ester (octocrylene) in combination with esters of cinnamic acid, preferably 4-methoxycinnamic acid-2-ethylhexyl ester and/or 4-methoxycinnamic acid propyl ester and/or 4-methoxycinnamic acid-isoamyl ester. Such combinations may preferably be combined with water-soluble filters such as 2-phenylbenzimidazole-5-sulfonic acid and alkali, alkaline earth, ammonium, alkylammonium, alkanolammonium, and glucammonium salts thereof.

Preferably the topical dermatological or cosmetic composition of the present invention comprises at least an additional UV absorbing substance selected from the group consisting of:
- 3-(4'-trimethylammonium)benzylidenebornan-2-one methyl sulphate
- homomenthyl salicylate (Neo Heliopan®HMS)
- terephthalylidenedibornanesulphonic acid and salts (Mexoryl®SX)
- 3-(4'-sulpho)benzylidenebornan-2-one and salts
- 2-ethylhexyl 2-cyano-3,3-diphenylacrylate (Neo Heliopan®303)
- N-[(2 and 4)-[2-(oxoborn-3-ylidene)methyl] benzyl]acrylamide polymer
- 2-ethylhexyl p-methoxycinnamate (Neo Heliopan®AV)
- ethyl p-aminobenzoate (25 mol) ethoxylated
- isoamyl p-methoxycinnamate (Neo Heliopan®E1000)
- 2-phenylbenzimidazole sulfonic acid (Neo Heliopan® Hydro) and its salts
- 2A6-trianilino(p-carbo-2'-ethylhexyl-1'-oxy)-1,3,5-triazine (Uvinul®T150)
- phenol,2-(2H-benzotriazol-2-yl)-4-methyl-6-(2-methyl-3(1,3,3,3-tetramethyl-1-(trimethylsilyl)oxy)disiloxyanyl)propyl), (Mexoryl®XL)
- 4,4'-[(6-[4-(1,1-dimethyl)aminocarbonyl)phenylamino]-1,3,5-triazin-2,4-diyl)-diimino] bis(benzoic acid 2-ethylhexyl ester), (Uvasorb® HEB)
- 3-(4'-methylbenzylidene)-d,1-camphor (Neo Helipan®M BC)
- 2-ethylhexyl salicylate (Neo Helipan®OS)
- 2-ethylhexyl 4-dimethylaminobenzoate (Padimate O)
- 4-hydroxy-4-methoxybenzophenone - 5-sulfonate (Benzophenone-4, Sulisobenzone) and its salts,
- benzylidenemalonate-polysiloxane (Parsol®SLX)
- menthyl anthranilate (Neo Heliopan®MA)
or mixtures thereof.

In addition to the above-mentioned filter substances also light protection pigments, specifically finely-dispersed metal oxides or salts, are suitable for UV protection purposes according to the present invention. Examples of particularly suitable metal oxides are zinc oxide and titanium dioxide, as well as iron, zirconium, silicon, manganese, aluminum, and cerium oxides and mixtures thereof. Silicates (talc), barium sulfate, or zinc stearate are particularly suitable salts. The oxides and salts are used in the form of pigments for skin care and skin protection emulsions and decorative cosmetics. In this case, the particles should have an average diameter of less than 100 nm, preferably 5 to 50 nm, and particularly preferably 15 to 30 nm. They preferably are spherical or ellipsoid in shape. The pigments may also be surface-treated, i.e. in a hydrophilized or hydrophobized form. Typical examples are coated titanium dioxides such as titanium dioxide T 805 (Degussa), Eusolex® T2000, Eusolex® T, Eusolex® T-ECO, Eusolex® T-S, Eu-solex® T-Aqua, Eusolex® T-45D (all Merck), and Uvinul TiO₂ (BASF). Examples of suitable hydrophobic coating agents are silicones, particularly trialkoxyoctyl silane or simethicone. Micropigments or nanopigments are preferably used in sun protection agents. Micronized zinc oxides such as Z-COTE® or Z-COTE HP1® are preferably used.

In a preferred embodiment the light protection pigment is selected from microfine titanium dioxide, zinc oxide, microfine zinc oxide. When titanium dioxide is chosen as the light protection pigment, it is advantageous that its total amount ranges from 0.1% to 10.0% by weight of the total composition. When zinc oxide is chosen as the light protection pigment it is advantageous that its total amount ranges from 0.1% to 10.0% by weight of the total composition, and when one or more triazine organic pigment(s) are chosen it is advantageous that its total amount ranges from 0.1% to 10.0% by weight based on the total composition.

Both the topical dermatologic composition and the cosmetic composition described in the following preferably contain carriers or solvents that are selected from the group selected from the group consisting of water, alcohols, esters, butylene glycol, dipropylene glycol, pentylene glycol, 1,2-hexane diol, caprylyl glycol, decylene glycol, ethanol, ethoxydiglycol, ethyl acetate, glycerol, propanol, isopropanol, macrogols, propyl propylene glycol(2) methyl ether, propyl propylene glycol(3) methyl ether, propylene carbonate, propylene glycol, triethylene glycol, iso-paraffin, amyl acetate, amyl benzoate, benzyl acetate, butyl acetate, butylene glycol, butyl lactate, butooctyl benzoate, butooctyl salicylate, C₁₀-C₁₃ alkanes, C₁₄-C₁₇ alkanes, C₁₁-C₁₅ cycloalkanes, caprylyl butyrate, isoparaffins, diacetin, triacetin dicaprylyl ether, dicaprylyl maleate, and mixtures thereof. Most preferred are glycerol, propylene glycol, butylene glycol, dipropylene glycol, pentylene glycol, 1,2-hexane diol, caprylyl glycol, decylene glycol.

The compositions in accordance with the present invention may contain further cosmetically and pharmaceutically/dermatologically acceptable excipients known to the skilled person. These include, next to the ones already mentioned above, for example further solvents such as organic solvents, carriers, gelling agents, detergents, emulsifiers, solubilizers, humectants, fillers, bioadhesives, emollients, preservatives, bactericides, surfactants, perfumes, thickeners, softening agents, moisturizing agents, oils, fats, waxes, water, alcohols, polyols, polymers, foam stabilizers, foaming agents, anti-foaming agents, hair coating agents or other suitable components of a pharmaceutical or cosmetic preparation. Perfumes are of particular importance in order to compensate the odor of vinegar (e.g. acetic acid) in the final compositions and products.

Examples for bactericides are organic acids like formic acid, sorbic acid, p-anisic acid and benzoic acid. In addition esters of p-hydroxybenzoic acid, formaldehyde-releasing agents like DMDM hydantoin, imidazolidinylurea or methyl chloroisothiazolinone, methylisothiazolinone, dibromodicyanobutane, iodopropynyl butylcarbamte, phenoxyethanol or benzyl alcohol can be used as bactericides.

Further, pharmaceutically/dermatologically acceptable excipients according to the present invention may be inorganic or organic substances for topical administration.

The compositions in accordance with the present invention may contain carriers or solvents that are selected from the group selected from the group consisting of water, alcohols, esters, butylene glycol, dipropylene glycol, pentylene glycol, 1,2-hexane diol, caprylyl glycol, decylene glycol, ethanol, ethoxydiglycol, ethyl acetate, glycerol, propanol, isopropanol, macrogols, propyl propylene glycol(2) methyl ether, propyl propylene glycol(3) methyl ether, propylene carbonate, propylene glycol, triethylene glycol, iso-paraffin, amyl acetate, amyl benzoate, benzyl acetate, butyl acetate, butylene glycol, butyl lactate, butooctyl benzoate, butooctyl salicylate, C₁₀-C₁₃ alkanes, C₁₄-C₁₇ alkanes, C₁₁-C₁₅ cycloalkanes, caprylyl butyrate, isoparaffins, diacetin, triacetin dicaprylyl ether, dicaprylyl maleate, and mixtures thereof. Most preferred are glycerol, propylene glycol, butylene glycol, dipropylene glycol, pentylene glycol, 1,2-hexane diol, caprylyl glycol, decylene glycol.

The compositions in accordance with the present invention may also contain further cosmetically and pharmaceutically/dermatologically active ingredients. Preferred active ingredients are plant (botanical) extracts such as all kinds of Echinacea extracts (e.g. an Echinacea extract disclosed in WO 2017/037075 such as an CO₂ extract of *Echinacea purpurea radix*) or ginger extracts (e.g. a ginger extract as disclosed in EP 2 772 245). A further particularly preferred additional active ingredient is caffeine.

In the present invention, the composition is preferably a dermatological or cosmetic composition suitable to be applied topically on the skin of a mammal. The form of the composition is not particularly limited. In preferred embodiments, the compositions are in the form of emulsions, lotions or creams (O/W and W/O, respectively), sprays, shampoos, serums, foams, facial masks, eye care products, cleansing products, or saturated pads. Suitable examples are facial or hand cremes with light protection (such as SPF), and cooling eye care products.

Clinical disorders/indications that may be treated (including treatment and/or prophylaxis) with the composition of the invention generally are all skin or hair diseases and conditions selected from the group consisting of oxidative stress related diseases (e.g. due to influences of temperature, nutrition, stress, lack of sleep, air/water pollution, tobacco, and/or alcohol), skin damages due to light radiation (e.g. sun light or artificial light, in particular UVB radiation) or detrimental environmental influences (e.g. toxic environment, air pollution), microbial skin infections, skin inflammations, raw skin, dry skin, irritated skin, itching, pruritus, allergies, psoriasis, psoriatic arthritis, eczema, scleroderma, atopic dermatitis, contact dermatitis, systemic lupus erythematosus, alopecia areata, androgenic alopecia including male pattern and female pattern baldness and chemotherapy-induced hair loss, acne and susceptibility to contact allergies.

Non-therapeutic (cosmetic) use of the composition of the present invention includes the topical application of the composition on the skin of a mammal in individuals for improving the epidermal barrier functions and the epidermal barrier integrity, for improving the appearance of skin or hair (anti-aging), for providing antioxidative effects and/or for increasing the moisture content of skin or hair. Cosmetic conditions of the skin or hair include oxidative stress related conditions (e.g. due to influences of temperature, nutrition, stress, lack of sleep, air/water pollution, tobacco, and/or alcohol), skin or hair conditions due to light radiation (e.g. sun light or artificial light, in particular UVB radiation), aging skin or hair, hair loss, hair graying, raw skin, dry skin, skin irritations, itching and pruritus, and for preventing skin infections and susceptibility to contact allergies.

Effects provided according to the invention include antioxidative effects, anti-aging effects, antimicrobial effects, antibacterial effects, fungicidal effects, and anti-inflammatory effects as well as anti-hair loss and anti-hair graying effects.

The composition of the present invention preferably is applied topically on the skin of a mammal. Typical application sites include the face/head (e.g. forehead, chin, neck and scalp), armpit, underarms, palms of the hands, soles of the feet, backs of the knees, trunk, and groin.

The composition of the present invention preferably is topically applied onto the skin of a mammal 1-4 times a day, more preferably 1-2 times a day, even more preferably 1 time per day prior to bedtime (for products without light protection).

### Brief description of the drawings:

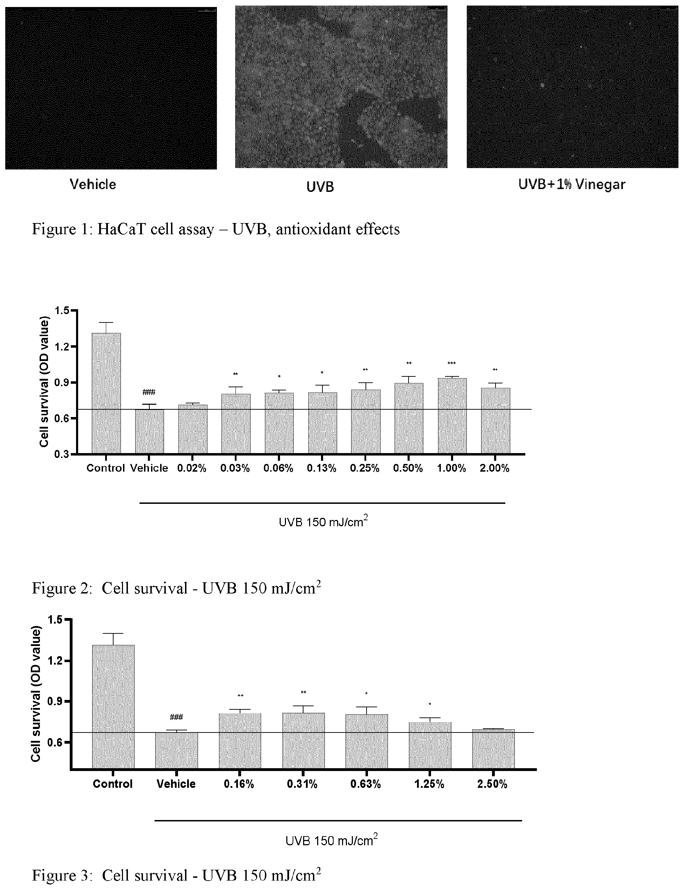

Figure 1 is a diagram that shows antioxidative effects of 1% litchi vinegar in HaCaT cells irradiated with UVB (150 mJ/cm²). Green fluorescence reflects the release of ROS.
Figure 2 is a diagram that shows cell survival in HaCaT cells following irradiation with UVB (150 mJ/cm²). Cells were treated with increasing concentration of litchi vinegar (pH not adjusted). ^{###} p<0.001 vs. control; * p<0.05, ** p <0.01, *** p<0.001 vs. vehicle.
Figure 3 is a diagram that shows cell survival in HaCaT cells following irradiation with UVB (150 mJ/cm²). Cells were treated with increasing concentration of litchi vinegar (pH adjusted). ^{###} p<0.001 vs. control; * p<0.05, ** p <0.01, *** p<0.001, **** p<0.0001 vs. vehicle.

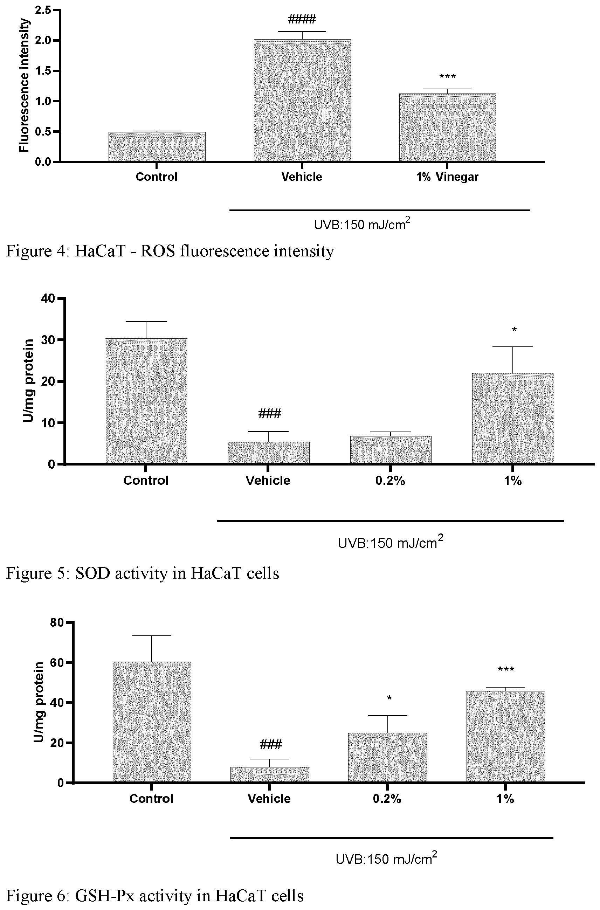

Figure 4 is a diagram that shows antioxidative effects of 1% litchi vinegar in HaCaT cells irradiated with UVB (150 mJ/cm²). ROS fluorescence was quantified. ^{####} p<0.0001 vs. control; *** p<0.001 vs. vehicle.
Figure 5 is a diagram that shows effects on SOD activity in HaCaT cells following irradiation with UVB (150 mJ/cm²). Litchi vinegar restored the reduction of SOD activity induced by UVB. ^{###} p<0.001 vs. control; * p<0.05 vs. vehicle.
Figure 6 shows effects on GSP-Px activity in HaCaT cells following irradiation with UVB (150 mJ/cm²). Litchi vinegar restored the reduction of GSP-Px activity induced by UVB. ^{###} p<0.001 vs. control; * p<0.05, *** p<0.001 vs. vehicle.

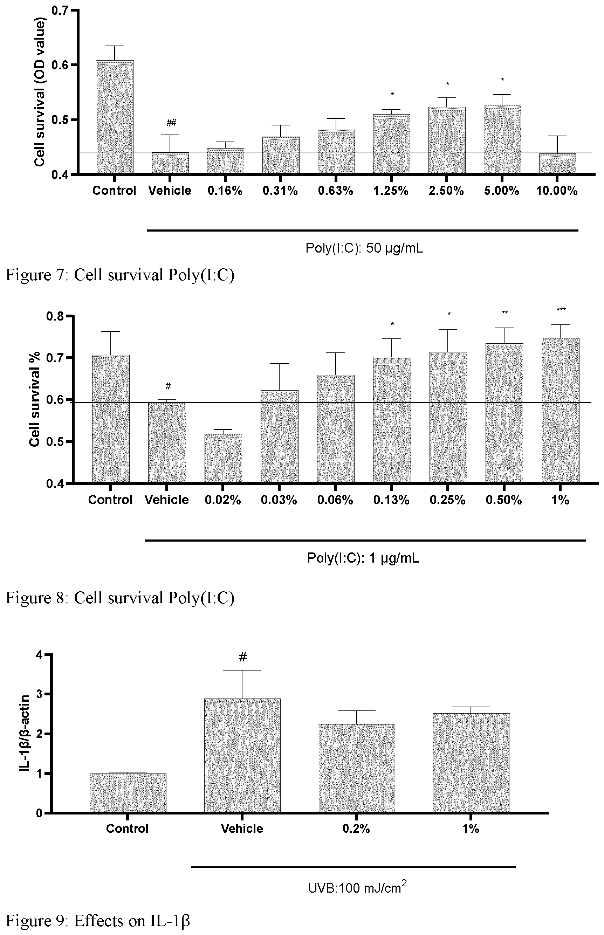

Figure 7 shows cell survival in HaCaT cells following stimulation with poly(I:C) (50 µg/mL). Cells were treated with increasing concentration of litchi vinegar (pH adjusted). ^{##} p<0.01 vs. control; * p<0.05 vs. vehicle.
Figure 8 shows cell survival in HaCaT cells following stimulation with poly(I:C) (1 µg/mL). Cells were treated with increasing concentration of litchi vinegar (pH not adjusted). ^{#} p<0.05 vs. control; * p<0.05, ** p<0.01, *** p<0.001 vs. vehicle.
Figure 9 shows effects on IL-1β mRNA expression in HaCaT cells following irradiation with UVB (100 mJ/cm²). Litchi vinegar treatment resulted in reduced expression of IL-1β compared to vehicle. ^{#} p<0.05 vs. control.

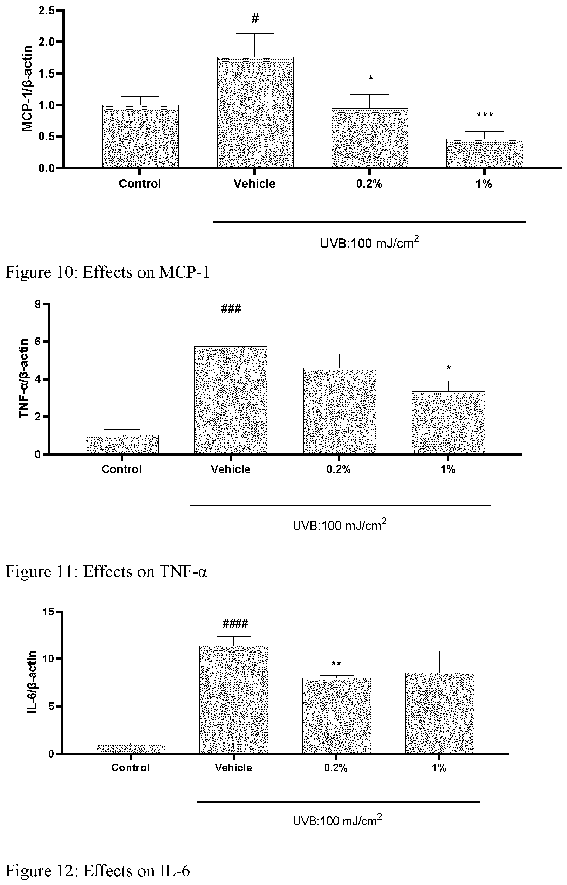

Figure 10 shows effects on MCP-1 mRNA expression in HaCaT cells following irradiation with UVB (100 mJ/cm²). Litchi vinegar treatment resulted in reduced expression of MCP-1 compared to vehicle. ^{#} p<0.05 vs. control; * p<0.05, *** p<0.001 vs. vehicle.
Figure 11 shows effects on TNF-α mRNA expression in HaCaT cells following irradiation with UVB (100 mJ/cm²). Litchi vinegar treatment resulted in reduced expression of TNF-α compared to vehicle. ^{#} p<0.05 vs. control; * p<0.05 vs. vehicle.
Figure 12 shows effects on IL-6 mRNA expression in HaCaT cells following irradiation with UVB (100 mJ/cm²). Litchi vinegar treatment resulted in reduced expression of IL-6 compared to vehicle. ^{####} p<0.0001 vs. control; ** p<0.01 vs. vehicle.

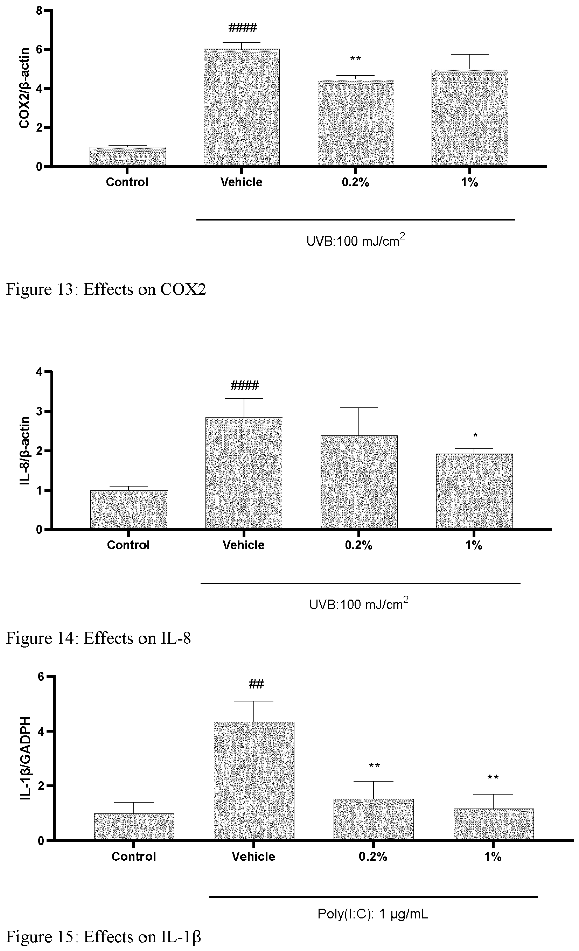

Figure 13 shows effects on COX-2 mRNA expression in HaCaT cells following irradiation with UVB (100 mJ/cm²). Litchi vinegar treatment resulted in reduced expression of COX-2 compared to vehicle. ^{####} p<0.0001 vs. control; ** p<0.01 vs. vehicle.
Figure 14 shows effects on IL-8 mRNA expression in HaCaT cells following irradiation with UVB (100 mJ/cm²). Litchi vinegar treatment resulted in reduced expression of IL-8 compared to vehicle. ^{####} p<0.0001 vs. control; * p<0.05 vs. vehicle.
Figure 15 shows effects on IL-1β mRNA expression in HaCaT cells following stimulation with poly(I:C) (1 µg/mL). Litchi vinegar treatment resulted in reduced expression of IL-1β compared to vehicle. ^{##} p<0.01 vs. control; ** p<0.01 vs. vehicle.

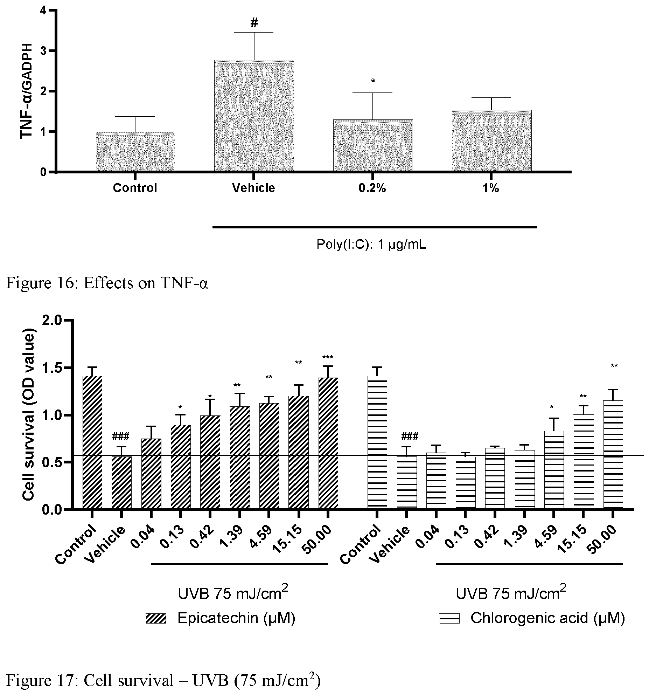

Figure 16 shows effects on TNF-α mRNA expression in HaCaT cells following stimulation with poly(I:C) (1 µg/mL). Litchi vinegar treatment resulted in reduced expression of TNF-α compared to vehicle. ^{#} p<0.05 vs. control; * p<0.05 vs. vehicle.
Figure 17 shows cell survival in HaCaT cells following irradiation with UVB (75 mJ/cm²). Cells were treated with increasing concentrations of epicatechin or chlorogenic acid. ^{###} p<0.001 vs. control; * p<0.05, ** p <0.01, *** p<0.001 vs. vehicle.

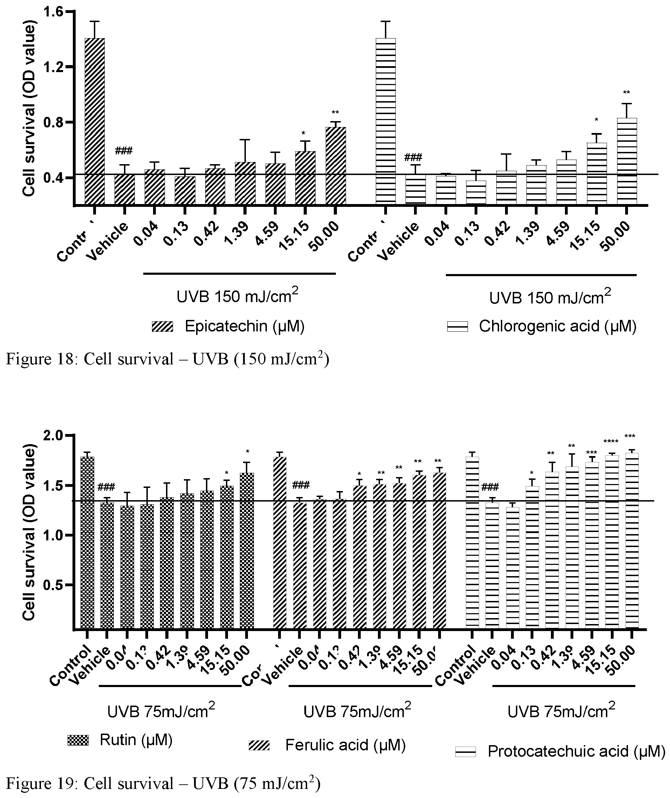

Figure 18 shows cell survival in HaCaT cells following irradiation with UVB (150 mJ/cm²). Cells were treated with increasing concentrations of epicatechin or chlorogenic acid. ^{###} p<0.001 vs. control; * p<0.05, ** p <0.01 vs. vehicle.
Figure 19 shows cell survival in HaCaT cells following irradiation with UVB (75 mJ/cm²). Cells were treated with increasing concentrations of rutin, ferulic acid or protocatechuic acid. ^{###} p<0.001 vs. control; * p<0.05, ** p <0.01, *** p<0.001, **** p<0.0001 vs. vehicle.

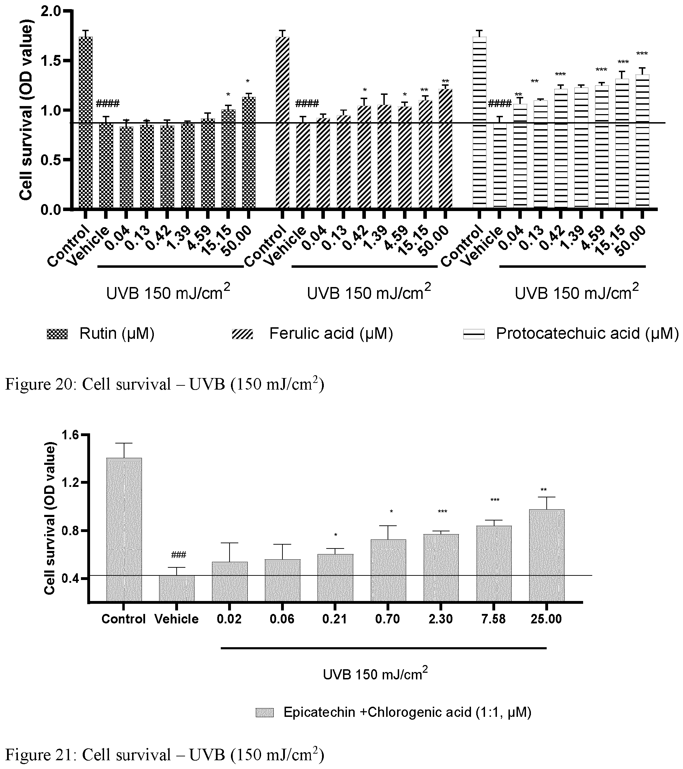

Figure 20 shows cell survival in HaCaT cells following irradiation with UVB (150 mJ/cm²). Cells were treated with increasing concentrations of rutin, ferulic acid or protocatechuic acid. ^{###} p<0.001 vs. control; * p<0.05, ** p <0.01, *** p<0.001 vs. vehicle.
Figure 21 shows cell survival in HaCaT cells following irradiation with UVB (150 mJ/cm²). Cells were treated with increasing concentrations of a mixture of epicatechin and chlorogenic acid (1:1). ^{###} p<0.001 vs. control; * p<0.05, ** p <0.01, *** p<0.001 vs. vehicle.

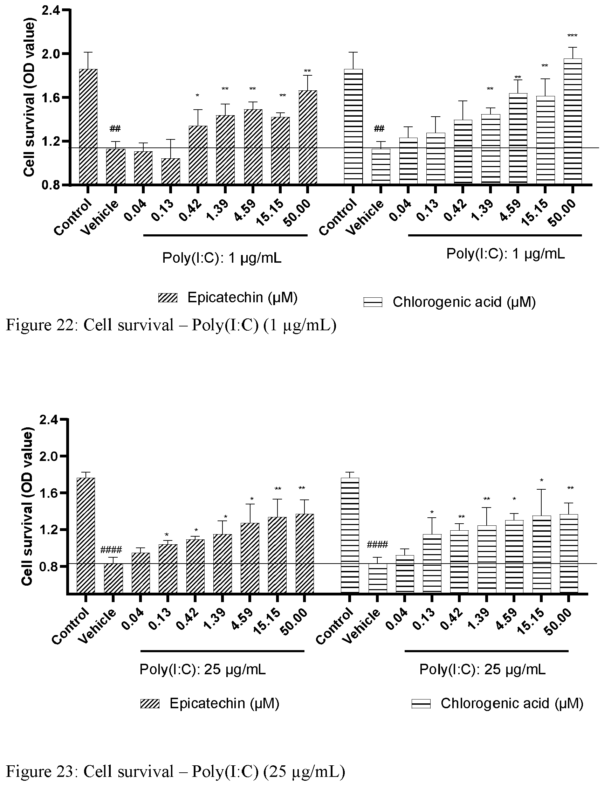

Figure 22 shows cell survival in HaCaT cells following stimulation with poly(I:C) (1 µg/mL). Cells were treated with increasing concentrations of epicatechin or chlorogenic acid (1:1). ^{##} p<0.01 vs. control; * p<0.05, ** p <0.01, *** p<0.001 vs. vehicle.
Figure 23 shows cell survival in HaCaT cells following stimulation with poly(I:C) (25 µg/mL). Cells were treated with increasing concentrations of epicatechin or chlorogenic acid (1:1). ^{####} p<0.0001 vs. control; * p<0.05, ** p <0.01 vs. vehicle.

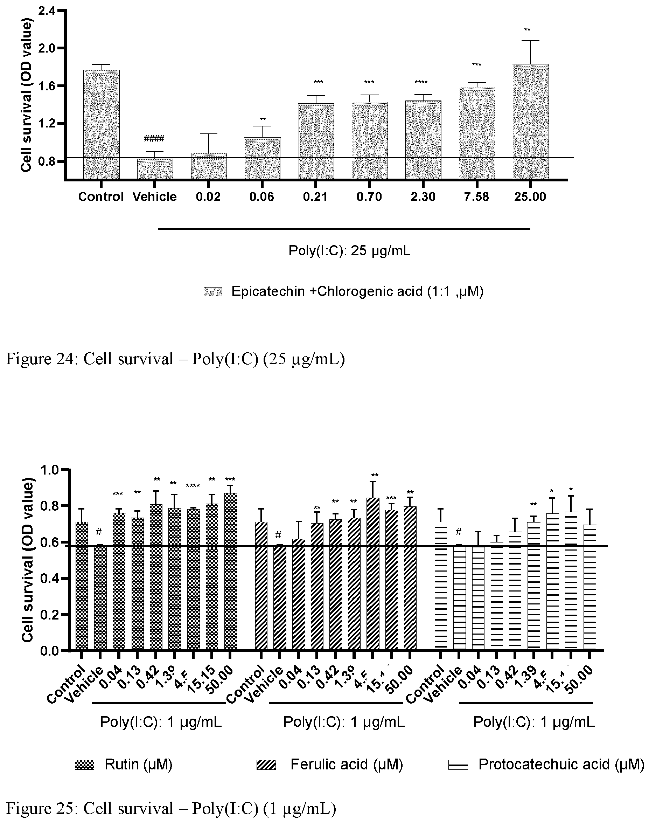

Figure 24 shows cell survival in HaCaT cells following stimulation with poly(I:C) (25 µg/mL). Cells were treated with increasing concentrations of a mixture of epicatechin and chlorogenic acid (1:1). ^{####} p<0.0001 vs. control; * p<0.05, ** p <0.01, *** p<0.001, **** p<0.0001 vs. vehicle.
Figure 25 shows cell survival in HaCaT cells following stimulation with poly(I:C) (1 µg/mL). Cells were treated with increasing concentrations of rutin, ferulic acid or protocatechuic acid. ^{#} p<0.05 vs. control; * p<0.05, ** p <0.01, *** p<0.001, **** p<0.0001 vs. vehicle.

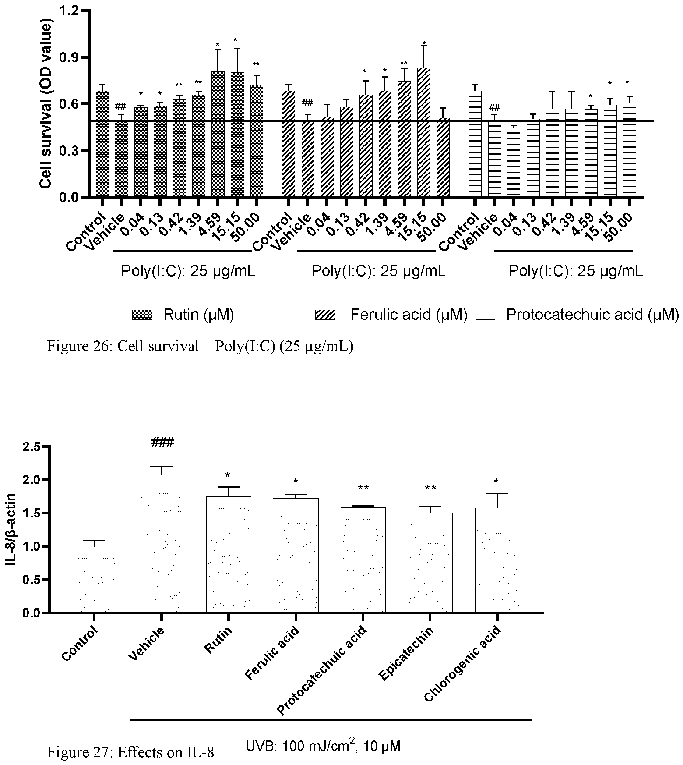

Figure 26 shows cell survival in HaCaT cells following stimulation with poly(I:C) (25 µg/mL). Cells were treated with increasing concentrations of rutin, ferulic acid or protocatechuic acid. ^{##} p<0.01 vs. control; * p<0.05, ** p <0.01 vs. vehicle.
Figure 27 shows effects on IL-8 mRNA expression in HaCaT cells following irradiation with UVB (100 mJ/cm²). Cells were treated with epicatechin, chlorogenic acid rutin, ferulic acid or protocatechuic acid (10 µM). Treatment resulted in reduced expression of IL-8 compared to vehicle. ^{###} p<0.001 vs. control; * p<0.05, ** p<0.01 vs. vehicle.

The following examples show preferred embodiments of the invention.

### Examples:

### A) Evaluation of Litchi Products - Summary

According to the present invention, there was evaluated litchi vinegar with assays on cell survival (OD, optical density), reactive oxygen species (ROS), superoxide dismutase (SOD), and glutathione peroxidase (GSH-Px) using keratinocytes (HaCaT cells), MTT assay, and skin irritation sources (UVB irradiation (dose: 75∼150 mJ/cm²) and poly(I:C) (dose: 1∼25 mg/mL) irritation). The biological targets were assayed against IL-1β, MCP-1, TNF-α, IL-6, COX-2, and IL-8. It was revealed that litchi vinegar (1) is not cytotoxic at therapeutic concentration; (2) protect skin cells against the irradiation caused by UVB or poly(I:C), significantly reduce the cell death caused by the irradiation and improve cell survival rate; (3) protect the cells against the oxidative stress caused by UVB or poly(I:C) irradiation through inhibiting ROS and enhancing SOD and GSH-Px activity; (4) the mechanisms of actions involving in suppressing inflammatory factor expressions including IL-1β, MCP-1, TNF-α, IL-6, COX-2, and IL-8; (5) Rutin, ferulic acid, protocatechuic acid, epicatechin, and chlorogenic acid are active ingredients from litchi vinegar, and protect the cells against the stresses caused by UVB irradiation and poly(I:C) irritation; (6) epicatechin and chlorogenic acid have better efficacy against UVB. (7) rutin has the best efficacy against poly(I:C) irritation followed by epicatechin and chlorogenic acid.

### B) Topical Compositions

Several topical compositions were prepared containing 1% or 2% by weight of the composition litchi vinegar (obtained from Guangdong Zhenzhou Litchi Industrial Co., Ltd., Huizhou, China). The compositions contain a perfume/fragrance to compensate the smell of vinegar (e.g. acetic acid). All these litchi products provide for combined antioxidative effects, antibacterial effects, fungicidal effects, anti-inflammatory effects, as well as skin care and skin protection effects.

**Night Moisturizer**

| | |
|---|---|
| Litchi vinegar | 1.00 g |
| Citric acid, anhydrous | 0.32 g |
| Sodium citrate | 0.26 g |
| Propylene glycol | 8.00 g |
| Phenoxyethanol | 1.00 g |
| Glycerin | 4.00 g |
| Sodium Polyacrylate | 0.50 g |
| Disodium EDTA | 0.05 g |
| Caprylic/Capric Triglyceride | 4.00 g |
| Cyclomethicone | 1.00 g |
| Dimethicone, 200 cs | 0.50 g |
| Cetearyl alcohol | 0.60 g |
| C₁₂-₂₂ alcohols | 0.40 g |
| C₁₂-₂₀ alkyl glucoside | 0.10 g |
| Perfume | 0.20 g |
| Water, purified | ad 100 g |
| | **100.000 g** |

**Night Moisturizer Intense**

| | |
|---|---|
| Litchi vinegar | 2.00 g |
| Citric acid, anhydrous | 0.32 g |
| Sodium citrate | 0.26 g |
| Chlorogenic acid | 0.25 g |
| Propylene glycol | 8.00 g |
| Phenoxyethanol | 1.00 g |
| Glycerin | 4.00 g |
| Sodium Polyacrylate | 0.50 g |
| Disodium EDTA | 0.05 g |
| Caprylic/Capric Triglyceride | 4.00 g |
| Cyclomethicone | 1.00 g |
| Dimethicone, 200 cs | 0.50 g |
| Cetearyl alcohol | 0.60 g |
| C₁₂-₂₂ alcohols | 0.40 g |
| C₁₂-₂₀ alkyl glucoside | 0.10 g |
| Perfume | 0.30 g |
| Water, purified | ad 100 g |
| | **100.000 g** |

**Day Lotion with light protecting agents**

| | |
|---|---|
| Litchi vinegar | 1.00 g |
| Citric acid, anhydrous | 0.32 g |
| Sodium citrate | 0.26 g |
| Propylene glycol | 2.00 g |
| Phenoxyethanol | 1.00 g |
| Disodium EDTA | 0.05 g |
| Acrylates copolymer | 2.00 g |
| Polyacrylamide | 0.06 g |
| Glyceryl stearate and PEG-100 stearate | 1.75 g |
| Cetyl alcohol | 1.75 g |
| C₁₃-₁₄ isoparaffin | 0.30 g |
| Laureth - 7 | 0.08 g |
| Cetearyl alcohol | 0.65 g |
| Ceteth-20 phosphate | 0.45 g |
| Dicetyl phosphate | 0.20 g |
| Homosalate | 10.0 g |
| Dimethicone, 200 cs. | 0.75 g |
| Salicylic acid-2-ethylhexyl ester | 5.00 g |
| 2-Hydroxy-4-methoxybenzophenone | 4.50 g |
| 1-(4-tert-Butylphenyl)-3-(4'-methoxyphenyl)propane-1,3-dione | 3.00 g |
| Perfume | 0.20 g |
| Water, purified | ad 100 g |
| | **100.000 g** |

## Claims

1. Litchi product for use as a medicament, preferably as a dermatological agent.

2. Litchi product for use according to claim 1, wherein the litchi product is selected from litchi wine and litchi vinegar.

3. Litchi product for use according to claim 1 and/or claim 2, wherein the medicament is for restoring the physiological pH of skin or hair and/or for protecting skin or hair against oxidative stress.

4. Litchi product for use according to any one of claims 1 to 3 for the therapeutic and/or prophylactic treatment of skin or hair diseases selected from the group consisting of oxidative stress related diseases, skin or hair damages due to light radiation or detrimental environmental influences, microbial skin infections, skin inflammations, raw skin, dry skin, irritated skin, itching, pruritus, allergies, psoriasis, psoriatic arthritis, eczema, scleroderma, atopic dermatitis, contact dermatitis, systemic lupus erythematosus, alopecia areata, androgenic alopecia including male pattern and female pattern baldness and chemotherapy-induced hair loss, acne and susceptibility to contact allergies.

5. Non-therapeutic use of a litchi product, preferably selected from litchi wine and litchi vinegar, as a cosmetic, preferably for restoring the physiological pH of skin or hair and/or for protecting skin or hair against oxidative stress and/or for preventing or reducing hair loss or hair graying.

6. The non-therapeutic use according to claim 5 for improving the epidermal barrier functions and the epidermal barrier integrity, for improving the appearance of skin or hair, for providing antioxidative effects and/or for increasing the moisture content of skin or hair.

7. The non-therapeutic use according to claim 5 and/or claim 6 for the treatment of cosmetic conditions of the skin or hair, selected from the group consisting of oxidative stress related conditions, skin and hair conditions due to light radiation, aging skin or hair, hair loss, hair graying, raw skin, dry skin, skin irritations, itching and pruritus, and for preventing skin infections and susceptibility to contact allergies.

8. Process for preparing the litchi product as defined in any one of claim 1 to 7, wherein the litchi product is litchi vinegar, and wherein the process comprises the steps of: harvesting litchi fruits, fruit sorting applying infrared technology, washing, peeling, pressing of pulps to obtain litchi juice, freeze-drying of juice, first fermentation, removal of alcohol, second fermentation.

9. Topical dermatological or cosmetic composition comprising a litchi product selected from litchi wine and litchi vinegar.

10. Topical dermatological or cosmetic composition according to claim 9, wherein the composition comprises the litchi product, preferably the litchi vinegar, in an amount of 0.1 to 10% by weight based on the total composition.

11. Topical dermatological or cosmetic composition according to claim 9 and/or claim 10, wherein the composition has a pH of 1 to 6, preferably of 2 to 5, more preferably of 3 to 5, even more preferably of 4 to 5.

12. Topical dermatological or cosmetic composition according to any one of claims 9 to 11, wherein the amount of acetic acid in the litchi product, preferably in litchi vinegar, is 3.5% by weight or more based on the weight of the litchi product.

13. Topical dermatological or cosmetic composition according to any one of claims 9 to 12, wherein at least one compound selected from the group of rutin, ferulic acid, protocatechuic acid, epicatechin, chlorogenic acid, caffeic acid, and gallic acid is further added to the composition.

14. Topical dermatological or cosmetic composition according to any one of claims 9 to 13, further comprising a buffering agent, preferably a citric acid/citrate buffer.

15. Topical dermatological or cosmetic composition according to any one of claims 9 to 14, further comprising at least one light filter compound.
